# EUROPEAN PATENT SPECIFICATION

(11) **EP 4 082 536 B1**
(45) Date of publication and mention of the grant of the patent: **09.04.2025**
(21) Application number: 20904953.5
(22) Date of filing: 18.12.2020
(51) Int. Cl.: A61K 9/30, A61K 31/195, A61P 25/00, A61K 9/28

(54) **FILM-COATED TABLETS HAVING SMOOTH SURFACE**
FILMÜBERZOGENE TABLETTEN, DIE EINE GLATTE OBERFLÄCHE AUFWEISEN
COMPRIMÉS PELLICULÉS AYANT UNE SURFACE LISSE

(30) Priority: 23.12.2019 JP 2019231824
(43) Date of publication of application: 02.11.2022
(73) Proprietor: Daiichi Sankyo Company, Limited, Tokyo 103-8426 (JP)
(72) Inventor: OZAKI Yurika, Tokyo 103-8426 (JP); YAMAGUCHI Minako, Tokyo 103-8426 (JP)
(74) Representative: Marks & Clerk LLP
(86) International application number: PCT/JP2020/047412
(87) International publication number: WO 2021/132072

(56) References cited:
- WO-A1-2014/174848
- WO-A1-2014/174848
- WO-A1-2016/148263
- WO-A1-2016/148264
- WO-A1-2020/027019
- US-A1- 2018 042 878
- US-A1- 2018 243 223

## Description

### Technical Field

The present invention relates to highly stable tablets containing mirogabalin or a pharmaceutically acceptable salt thereof.

The tablets of the present invention are film-coated tablets, and the surface of the tablets is very smooth even after storage at high humidity in an unpackaged state, with substantially no visible dimples found thereon.

The present invention also relates to a method for producing the same.

### Background Art

As dosage forms of solid preparations for oral administration in the fields of pharmaceuticals and food, tablets, capsules, granules, powders and the like are known. Particularly regarding tablets, patients are more likely to feel secure and trust clean tablets with good visual quality free from defects such as a dimples, chips, or black spots, even if the actual quality is equivalent, and thus such good visual quality is important.

Patent Literature 1 describes a solid composition for medical use, containing mirogabalin besylate, (i) one selected from the group consisting of D-mannitol, lactose, corn starch and crystalline cellulose, and (ii) carmellose calcium. This document does not, however, describe tablets containing mirogabalin besylate and having a smooth surface.

Patent Literature 2 discloses that mirogabalin besylate is stabilized in a solid preparation for medical use containing mirogabalin besylate together with an excipient, a disintegrating agent, and a specific antioxidant. This document does not, however, describe tablets containing mirogabalin besylate and having a smooth surface.

Patent Literature 3 describes a solid composition for medical use, containing mirogabalin besylate together with (i) one or two or more selected from the group consisting of D-mannitol, lactose, corn starch, and crystalline cellulose, (ii) carmellose calcium, and (iii) titanium oxide used as a colorant, and one or two or more other colorants. This document does not, however, describe tablets containing mirogabalin besylate and having a smooth surface.

Patent Literature 4 discloses methods for suppressing the occurrence of spots on the surface of coated tablets containing an acidic substance such as citric acid.

### Citation List

### Patent Literature

Patent Literature 1: US2015/0079166A1
Patent Literature 2: US2018/0042878A1
Patent Literature 3: US2018/0243223A1
Patent Literature 4: WO2014/178848A1

### Summary of Invention

### Technical Problem

A problem to be solved by the present invention is to provide high-quality tablets containing mirogabalin or a pharmaceutically acceptable salt thereof.

Tablets of the present invention are film-coated tablets, and the surface of the tablets is very smooth even after storage at high humidity in an unpackaged state, with substantially no visible dimples found thereon.

The present inventors have found that tablets containing mirogabalin besylate produced by a conventional method have no problem in quality and safety, but that small dimples (usually having a length of about 100 to 300 µm) are formed on the surface of the tablets.

These small dimples do not affect the original function as a pharmaceutical at all, but a patient taking the tablets in his/her hand may care about the dimples, and therefore, examinations have been made on tablets having no dimples on their surface, and having a very smooth surface with no visible dimples found thereon.

As a result, it was found that the problem can be solved by adjusting the particle size of citric acid anhydride or citric acid hydrate contained as an ingredient of the tablets, and thus, the present invention was accomplished.

### Solution to Problem

The present inventors have made diligent studies to solve the above-described problem, thereby accomplishing the present invention and providing highly stable film-coated tablets containing mirogabalin or a pharmaceutically acceptable salt thereof, and having a very smooth surface with substantially no visible dimples found thereon.

Specifically, the present invention provides the invention described below:
[1] A film-coated tablet obtained by film coating an uncoated tablet containing citric acid anhydride having a particle size distribution x99 of less than 210 µm or citric acid hydrate having a particle size distribution x99 of less than 210 µm, and α-tocopherol.
[2] A film-coated tablet obtained by film coating an uncoated tablet containing: mirogabalin or a pharmaceutically acceptable salt thereof; and citric acid anhydride having a particle size distribution x99 of less than 210 µm or citric acid hydrate having a particle size distribution x99 of less than 210 µm, and α-tocopherol.
[3] The film-coated tablet according to [2], wherein mirogabalin or a pharmaceutically acceptable salt thereof is mirogabalin besylate.
[4] The film-coated tablet according to any one of [1] to [3], wherein the content of the citric acid anhydride having a particle size distribution x99 of less than 210 µm or the citric acid hydrate having a particle size distribution x99 of less than 210 µm is, in terms of a citric acid hydrate, 1.0 to 5.0% by weight based on 100% by weight of the uncoated tablet.
[5] The film-coated tablet according to any one of [1] to [3], wherein the content of the citric acid anhydride having a particle size distribution x99 of less than 210 µm or the citric acid hydrate having a particle size distribution x99 of less than 210 µm is, in terms of a citric acid hydrate, 1.125 to 2.9% by weight based on 100% by weight of the uncoated tablet.
[6] The film-coated tablet according to any one of [1] to [5], wherein the particle size distribution x99 of the citric acid anhydride or the citric acid hydrate is less than 150 µm.
[7] The film-coated tablet according to any one of [1] to [6], wherein the content of α-tocopherol is 0.005 to 1.0% by weight based on 100% by weight of the uncoated tablet.
[8] The film-coated tablet according to any one of [1] to [6], wherein the content of α-tocopherol is 0.005 to 0.5% by weight based on 100% by weight of the uncoated tablet.
[9] The film-coated tablet according to any one of [3] to [7], wherein the content of mirogabalin besylate is, in terms of mirogabalin, 1.0 to 5.0% by weight based on 100% by weight of the uncoated tablet.
[10] The film-coated tablet according to any one of [1] to [9], further containing 75 to 85% by weight of D-mannitol having an average particle size of 120 µm or less based on 100% by weight of the uncoated tablet.
[11] The film-coated tablet according to any one of [1] to [10], further containing 5 to 15% by weight of carmellose calcium based on 100% by weight of the uncoated tablet.
[12] A method for producing a film-coated tablet obtained by film coating an uncoated tablet containing mirogabalin or a pharmaceutically acceptable salt thereof, the method comprising: (i) a step of producing an uncoated tablet containing mirogabalin or a pharmaceutically acceptable salt thereof, citric acid anhydride having a particle size distribution x99 of less than 210 µm or citric acid hydrate having a particle size distribution x99 of less than 210 µm, and α-tocopherol; and (ii) a step of film coating the uncoated tablet obtained in (i).

### Advantageous Effect of Invention

The present invention is able to provide highly stable film-coated tablets containing mirogabalin or a pharmaceutically acceptable salt thereof, in which the surface of the tablets is very smooth even after storage at high humidity in an unpackaged state, with substantially no visible dimples found thereon, and which is obtained by adjusting the particle size of the citric acid anhydride or citric acid hydrate contained therein.

### Description of Embodiments

"Mirogabalin" used in the present invention is a compound represented by the following formula (I):

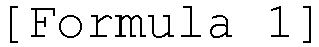

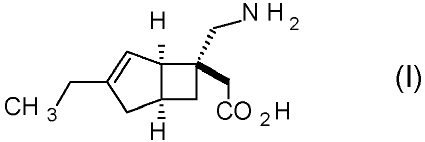

"Mirogabalin besylate" used in the present invention is a salt formed from mirogabalin and besylic acid, and is a salt represented by the following formula (Ia):

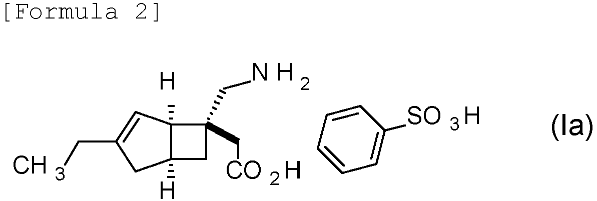

The term "pharmaceutically acceptable salt" used in the present invention refers to a salt which can be used as a pharmaceutical. Usually, when a compound has an acidic group or a basic group, a base addition salt or an acid addition salt is generated through a reaction with a base or an acid, and this term refers to such a salt.

"Mirogabalin" used in the present invention is considered to exhibit an analgesic effect by suppressing calcium current by binding to the α2δ subunit, which plays an auxiliary role in the function of voltage-dependent calcium channels in the nervous system.

"Mirogabalin besylate" used in the present invention has been approved for manufacturing and marketing as a therapeutic agent for peripheral neuropathic pain through clinical trials conducted in Japan and overseas, and is commercially available.

In treatment of peripheral neuropathic pain, usually, an initial dose of 5 mg, in terms of mirogabalin, is orally administered twice a day for adults, and thereafter, the dose is gradually increased by 5 mg at intervals of 1 week or more to orally administer a dose of 15 mg twice a day. It is noted that the dose is appropriately increased/decreased in a range of 10 mg to 15 mg per administration depending on the age and symptoms, so as to be administered twice a day.

Mirogabalin besylate used in the present invention has an average particle size of preferably 60 µm (more preferably 40 µm) or less.

The term "average particle size" used in the present invention means the particle size at which a volume based cumulative frequency obtained by a laser diffraction/scattering method is 50%.

The content of mirogabalin besylate used in the present invention is, in terms of mirogabalin, preferably 0.5 to 40% by weight, more preferably 0.5 to 25% by weight, and particularly preferably 0.5 to 10% by weight (more particularly preferably 1.0 to 5.0% by weight) based on 100% by weight of an uncoated tablet.

The term "dimple" used in the present invention indicates a state where a fine pit is formed on a smooth surface of film-coated tablets.

A dimple does not penetrate through a film coating layer, and the film-coated tablet is wholly covered with the film coating layer even when it has the dimples. As for the size of a dimple, the length and the width are both about 50 µm to 300 µm, and the depth is about 50 µm at most.

According to the present invention, film-coated tablets having substantially no visible dimples found thereon can be produced. Preferably, film-coated tablets having no visible dimples found thereon can be produced.

The term "particle size distribution" used in the present invention is an index indicating, in a sample particle group to be measured, particles having which particle size are contained in which ratio (relative amount of particles assuming that the total amount is 100%), and has the same meaning as the "grain size distribution".

The term "particle size distribution x50" used in the present invention means the particle size at which the volume based cumulative frequency obtained by a laser diffraction/scattering method is 50%.

The term "particle size distribution x90" used in the present invention means the particle size at which the volume based cumulative frequency obtained by the laser diffraction/scattering method is 90%.

The term "particle size distribution x99" used in the present invention means the particle size at which the volume based cumulative frequency obtained by the laser diffraction/scattering method is 99%.

The content of "D-mannitol" used in the present invention is usually 50 to 90% by weight, and preferably 75 to 85% by weight based on 100% by weight of the uncoated tablet.

The average particle size of D-mannitol used in the present invention is preferably smaller than 150 µm, and preferably 120 µm or less.

D-mannitol in powder form may be mixed using other ingredients to obtain a tableting powder to be compression molded, or may be compression molded after the powder is granulated with an appropriate binding agent.

The content of "carmellose calcium" used in the present invention is usually 2.0 to 20% by weight, and preferably 5.0 to 15% by weight based on 100% by weight of the uncoated tablet.

The content of "magnesium stearate" used in the present invention is usually 0.5 to 5.0% by weight, and preferably 1.0 to 3.0% by weight based on 100% by weight of the uncoated tablet.

The "citric acid anhydride or citric acid hydrate" used in the present invention is preferably citric acid hydrate. The content of the "citric acid anhydride or citric acid hydrate" used in the present invention is, in terms of a citric acid hydrate, usually 0.01 to 10.0% by weight, preferably 1.0 to 5.0% by weight, more preferably 1.125 to 2.9% by weight, and particularly preferably 1.5 to 2.9% by weight based on 100% by weight of the uncoated tablet.

The particle size distribution x99 of the "citric acid anhydride or citric acid hydrate" used in the present invention is preferably less than 210 µm, more preferably less than 200 µm, and particularly preferably less than 150 µm.

The content of "α-tocopherol" used in the present invention is usually 0.005 to 1.0% by weight, and preferably 0.05 to 0.5% by weight based on 100% by weight of the uncoated tablet.

The content of "crystalline cellulose" used in the present invention is preferably 0.1 to 50% by weight based on 100% by weight of the uncoated tablet. A more preferred blending amount is 0.9 to 4.5% by weight.

The film-coated tablets used in the present invention can contain various "additives" generally used in the production of tablets unless the effect of the invention is impaired.

Examples of "additives" include a binding agent, a lubricant, a coating agent, a plasticizer, a colorant, a flavoring, a sweetening agent, a taste masking agent, a fluidizer, a foaming agent, and a surfactant.

Examples of the "binding agent" include one or a combination of two or more selected from gum arabic, sodium alginate, a carboxyvinyl polymer, gelatin, dextrin, pectin, sodium polyacrylate, pullulan, methylcellulose, hydroxypropyl cellulose, hydroxypropyl methylcellulose, polyvinyl alcohol, polyvinyl pyrrolidone, and macrogol.

Examples of the "lubricant" include one or a combination of two or more selected from magnesium stearate (for example, a product conforming to the Japanese Pharmacopoeia), calcium stearate (for example, a product conforming to the Japanese Pharmacopoeia), stearyl sodium fumarate (for example, a product conforming to the standards of Japanese Pharmaceutical Excipients), and talc (for example, a product conforming to the Japanese Pharmacopoeia), and particularly preferably magnesium stearate.

With regard to the "coating agent", examples of coating agents for coating the surface (crystal surface) of a powdered drug or a granule surface of a granulated drug include one or a combination of two or more selected from hydroxypropyl cellulose, hydroxypropyl methylcellulose, ethylcellulose, amino alkyl methacrylate copolymer E, methacrylic acid copolymer L, dry methacrylic acid copolymer LD, methacrylic acid copolymer LD, methacrylic acid copolymer S, amino alkyl methacrylate copolymer RS, an ethyl acrylate/methyl methacrylate copolymer, polyvinyl acetal/diethyl aminoacetate, and a polyvinyl acetate resin.

The "plasticizer" is one conventionally used in combination with a coating agent, and examples include one or a combination of two or more selected from diethyl sebacate, dibutyl sebacate, triethyl citrate, stearic acid, polyethylene glycol, and triacetin.

Examples of the "colorant" include one or a combination of two or more selected from food coloring such as food yellow No. 5, food red No. 2, or food blue No. 2; and food lake colors, yellow iron sesquioxide, iron sesquioxide, titanium oxide, β-carotene, and riboflavin.

Examples of the "flavoring" include one or a combination of two or more selected from orange, lemon, strawberry, mint, menthol, Menthol Micron, and other various flavorings.

Examples of the "sweetening agent" include one or a combination of two or more selected from saccharin sodium, saccharin, aspartame, acesulfame potassium, dipotassium glycyrrhizate, sucralose, stevia, and thaumatin.

Examples of the "taste masking agent" include one or a combination of two or more selected from sodium chloride, magnesium chloride, disodium inosinate, sodium L-glutamate, and honey.

Examples of the "fluidizer" include one or a combination of two or more selected from hydrated silicon dioxide, light anhydrous silicic acid, and talc.

Examples of the "foaming agent" include tartaric acid and/or citric acid anhydride.

Examples of the "surfactant" include one or a combination of two or more selected from polyoxyl 40 stearate, sorbitan fatty acid ester, polyoxyethylene hydrogenated castor oil, polysorbate, glycerin monostearate, and sodium lauryl sulfate.

### (Method for Producing Solid Preparation)

Examples of methods for producing tablets include:
(1) a direct compression method in which an active ingredient and an additive are mixed, and the resultant mixture is directly compression molded using a tablet press;
(2) a semi-direct compression method in which an additive is granulated to be mixed using an active ingredient, and the resultant is compression molded;
(3) a dry granule compression method in which an active ingredient and an additive are granulated into granules by a dry method, a lubricant and the like are added thereto, and the resultant is compression molded; and
(4) a wet granule compression method in which an active ingredient and an additive are granulated into granules by a wet method, a lubricant and the like are added thereto, and the resultant is compression molded.

As a granulation method, means such as a fluidized granulation method, a high shear granulation method, or melting granulation method can be employed.

In the present invention, a method for preparing tablets by granulating certain additives without granulating the powder of the active ingredient, and directly compressing the mixed powder of these, is preferred.

For example, a method for producing tablets of the present invention is as follows:
The active ingredient is pulverized to adjust the particle size, and an excipient and/or a disintegrating agent are added thereto to be mixed. Besides, a mixed triturated powder is prepared by adding, to a stabilizing agent, an excipient and/or a disintegrating agent if necessary. For example, when the stabilizing agent is an oily substance such as α-tocopherol, a mixed triturated powder is preferably prepared by adding an excipient such as crystalline cellulose in advance.

Thereafter, a mixture of all ingredients is sifted using a granulator, a lubricant is added to the resultant to be further mixed, and the resultant is compressed using a tablet press to obtain uncoated tablets.

The thus obtained uncoated tablets are formed into film-coated tablets using a coating machine.

### Examples

The present invention will be described more specifically with reference to examples and the like, but the following examples are merely illustrative for describing the present invention, and it should not be construed that the present invention is limited to these examples.

### (Examples 1 to 6) Examination of Cause of Appearance Change in High Humidity Storage

### (1) Example 1

### (Trituration Mixing)

A premixed powder was obtained by sufficiently mixing 9.4 g of dl-α-tocopherol and 62.6 g of carmellose calcium in a mortar. 70.3 g of the premixed powder and 244.7 g of D-mannitol were put in a polyethylene bag to be mixed, and the resultant was sifted through a 500 µm mesh to obtain a dl-α-tocopherol mixed triturated powder.

### (Mixing/sifting)

Mirogabalin besylate, D-mannitol, citric acid hydrate, and the dl-α-tocopherol mixed triturated powder were weighed out in the blending ratio shown in Table 1, and the resultant mixture was mixed using a V-type mixer (2 L) for 5 minutes at a rotation speed of 39 rpm.

The resultant was sifted using a Comil (U-5, φ1.143, QUADRO) at 600 rpm to obtain a sifted powder.

Subsequently, magnesium stearate was weighed out in the blending ratio shown in Table 1 to be added to the sifted powder, and the resultant was mixed using a V-type mixer (2 L) for 5 minutes at a rotation speed of 39 rpm.

### (Compressing)

The resultant was molded using a tablet press (Vela 5, Kikusui Seisakusho Ltd.) with the tablet mass set to 100 mg at a compression pressure of about 7.5 kN to obtain uncoated tablets (containing mirogabalin in an amount of 2.5% by weight with respect to the uncoated tablet, oblong tablet, 8.4 x 4.4 mm).

### (Coating)

A stirrer (MAZELA Z, Tokyo Rikakikai Co., Ltd.) was used to disperse OPADRY (R) (which herein refers to a mixture of hypromellose, talc, titanium oxide, iron sesquioxide, and yellow iron sesquioxide) in purified water (12.5 w/w%) to obtain a coating liquid.

The uncoated tablets were coated using a coating machine (Hicoater FZ20, Freund Corp.) at a charge air temperature of 75°C, a charge air flow rate of 0.5 m³/min, a spray rate of about 3.5 g/min, a pan rotation speed of 25 rpm, and an exhaust gas drying end point of about 60°C to obtain coated tablets.

### (2) Example 2

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 5 minutes.

### (Mixing/sifting)

Mirogabalin besylate, D-mannitol, carmellose calcium, citric acid hydrate, the dl-α-tocopherol mixed triturated powder, and magnesium aluminometasilicate were weighed out in the blending ratio shown in Table 1, and the resultant mixture was mixed using a V-type mixer (5 L) for 10 minutes at a rotation speed of 34 rpm.

The resultant was sifted using a Comil (QC-194S, φ1.143, QUADRO) at 600 rpm to obtain a sifted powder.

Subsequently, magnesium stearate was weighed out in the blending ratio shown in Table 1 to be added to the sifted powder, and the resultant was mixed using a V-type mixer (5 L) for 7 minutes at a rotation speed of 34 rpm.

### (Compressing)

The resultant was molded using a tablet press (Virgo, Kikusui Seisakusho Ltd.) with the tablet mass set to 200 mg at a compression pressure of about 10 kN to obtain uncoated tablets (containing mirogabalin in an amount of 2.5% by weight with respect to the uncoated tablet, oblong tablet, 10.6 x 5.6 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated using a coating machine (Hicoater LABO 30, Freund Corp.) at a charge air temperature of 70°C, a charge air flow rate of 0.8 m³/min, a spray rate of about 8 g/min, a pan rotation speed of 20 rpm, and an exhaust gas drying end point of about 60°C to obtain coated tablets.

### (3) Example 3

In Example 3, coated tablets were prepared by using respective ingredients and their contents shown in Table 1 by the same preparation method as that of Example 1.

### (4) Example 4

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 25 minutes.

### (Sifting of Citric Acid Hydrate)

Citric acid hydrate was manually sifted through a 200 mesh (having an opening of 75 µm) to obtain a citric acid hydrate sifted powder.

### (Mixing/sifting)

Mirogabalin besylate, D-mannitol, carmellose calcium, the citric acid hydrate sifted powder, the dl-α-tocopherol mixed triturated powder, and magnesium aluminometasilicate were weighed out in the blending ratio shown in Table 1, and the resultant mixture was mixed using a V-type mixer (2 L) for 10 minutes at a rotation speed of 39 rpm.

The resultant was sifted using a Comil (U-5, φ1.143, QUADRO) at 1560 rpm to obtain a sifted powder.

Subsequently, magnesium stearate was weighed out in the blending ratio shown in Table 1 to be added to the sifted powder, and the resultant was mixed using a V-type mixer (2 L) for 7 minutes at a rotation speed of 39 rpm.

### (Compressing)

The resultant was molded using a tablet press (Vela 2, Kikusui Seisakusho Ltd.) with the tablet mass set to 300 mg at a compression pressure of about 15 kN to obtain uncoated tablets (oblong tablet, 12.1 x 6.4 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated with a coating machine (Hicoater FZ 20, Freund Corp.) at a charge air temperature of 70°C, a charge air flow rate of 0.5 m³/min, a spray rate of about 2 g/min, a pan rotation speed of 20 rpm, and an exhaust gas drying end point of about 54°C to obtain coated tablets.

### (5) Example 5

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 25 minutes.

### (Premixing of Citric Acid Hydrate)

A citric acid hydrate premixed powder was obtained by mixing 754.2 g of D-mannitol and 33.0 g of citric acid hydrate using a high-speed agitation granulator (VG-5L, Powrex Corp.) at an agitator rotation speed of 280 rpm and a chopper rotation speed of 3000 rpm for 15 minutes.

### (Mixing/sifting)

The citric acid hydrate premixed powder, D-mannitol, carmellose calcium, the dl-α-tocopherol mixed triturated powder, and magnesium aluminometasilicate were weighed out in the blending ratio shown in Table 1, and the resultant mixture was mixed using a V-type mixer (2 L) for 10 minutes at a rotation speed of 39 rpm.

The resultant was sifted using a Comil (QC-194S, φ0.457, QUADRO) at 1700 rpm to obtain a sifted powder.

Subsequently, magnesium stearate was weighed out in the blending ratio shown in Table 1 to be added to the sifted powder, and the resultant was mixed using a V-type mixer (2 L) for 7 minutes at a rotation speed of 39 rpm.

### (Compressing)

The resultant was molded using a tablet press (Vela 2, Kikusui Seisakusho Ltd.) with the tablet mass set to 300 mg at a compression pressure of about 15 kN to obtain uncoated tablets (oblong tablet, 12.1 x 6.4 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated with a coating machine (Hicoater FZ 20, Freund Corp.) at a charge air temperature of 70°C, a charge air flow rate of 0.5 m³/min, a spray rate of about 2 g/min, a pan rotation speed of 20 rpm, and an exhaust gas drying end point of about 54°C to obtain coated tablets.

### (6) Example 6

### (Pulverizing Citric Acid Hydrate)

Citric acid hydrate was pulverized with a fine impact mill (100 UPZ, Hosokawa Micron Corporation) at a disc rotation speed of 16000 rpm to obtain a citric acid hydrate pulverized powder.

Thereafter, the citric acid hydrate pulverized powder was used in processes from trituration mixing to coating which were performed as in the preparation method of Example 5. Respective ingredients and their contents are shown in Table 1.

### (7) Comparative Example 1

### (Trituration Mixing)

A premixed powder was obtained by sufficiently mixing 9.4 g of dl-α-tocopherol and 62.6 g of carmellose calcium in a mortar. 70.3 g of the premixed powder and 244.7 g of D-mannitol were put in a polyethylene bag to be mixed, and the resultant was sifted through a 500 µm mesh to obtain a dl-α-tocopherol mixed triturated powder.

### (Mixing/sifting)

Mirogabalin besylate, D-mannitol, citric acid hydrate, and the dl-α-tocopherol mixed triturated powder were weighed out in the blending ratio shown in Table 1, and the resultant mixture was mixed using a V-type mixer (2 L) for 5 minutes at a rotation speed of 39 rpm.

The resultant was sifted using a Comil (U-5, φ1.143, QUADRO) at 600 rpm to obtain a sifted powder.

Subsequently, magnesium stearate was weighed out in the blending ratio shown in Table 1 to be added to the sifted powder, and the resultant was mixed using a V-type mixer (2 L) for 5 minutes at a rotation speed of 39 rpm.

### (Compressing)

The resultant was molded using a tablet press (Vela 5, Kikusui Seisakusho Ltd.) with the tablet mass set to 100 mg at a compression pressure of about 7.5 kN to obtain uncoated tablets (containing mirogabalin in an amount of 2.5% by weight with respect to the uncoated tablet, oblong tablet, 8.4 x 4.4 mm).

### (8) Comparative Example 2

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 5 minutes.

### (Mixing/sifting)

Mirogabalin besylate, D-mannitol, carmellose calcium, citric acid hydrate, the dl-α-tocopherol mixed triturated powder, and magnesium aluminometasilicate were weighed out in the blending ratio shown in Table 1, and the resultant mixture was mixed using a V-type mixer (5 L) for 10 minutes at a rotation speed of 34 rpm.

The resultant was sifted using a Comil (QC-194S, φ1.143, QUADRO) at 600 rpm to obtain a sifted powder.

Subsequently, magnesium stearate was weighed out in the blending ratio shown in Table 1 to be added to the sifted powder, and the resultant was mixed using a V-type mixer (5 L) for 7 minutes at a rotation speed of 34 rpm.

### (Compressing)

The resultant was molded using a tablet press (Virgo, Kikusui Seisakusho Ltd.) with the tablet mass set to 200 mg at a compression pressure of about 10 kN to obtain uncoated tablets (containing mirogabalin in an amount of 2.5% by weight with respect to the uncoated tablet, oblong tablet, 10.6 x 5.6 mm).

**[Table 1]**

| | Composition (wt%/uncoated tablet) | | | | | | | |
|---|---|---|---|---|---|---|---|---|
| Ingredients | Example 1 | Example 2 | Example 3 | Example 4 | Example 5 | Example 6 | Comparative Example 1 | Comparative Example 2 |
| Mirogabalin Besylate (in terms of mirogabalin) | 4.39 (2.5) | 4.39 (2.5) | 4.39 (2.5) | 8.78 (5) | - | - | 4.39 (2.5) | 4.39 (2.5) |
| D-mannitol (Parteck M100, Merck, Mannogem EZ, SPI Pharma) | 80.61 | 79.11 | 82.11 | 76.92 | 85.70 | 85.70 | 80.61 | 79.11 |
| Carmellose Calcium (E.C.G-505, Gotoku Chemical Co., Ltd.) | 10 | 10 | 10 | 10 | 10 | 10 | 10 | 10 |
| dl-α-Tocopherol (Mitsubishi Chemical Foods Corp.) | 1.5 | 0.19 | 1.5 | 0.1 | 0.1 | 0.1 | 1.5 | 0.19 |
| Crystalline Cellulose (Ceolus UF-702, Asahi Kasei Corp.) | - | 1.7 | - | 0.9 | 0.9 | 0.9 | - | 1.7 |
| Citric Acid Hydrate (Merck) | 1.5 | 2.81 | - | 1.5 ¹⁾ | 1.5 | 1.5 ²⁾ | 1.5 | 2.81 |
| Magnesium Aluminometasilicate (Neusilin UFL2, US2, Fuji Chemical Industries, Co., Ltd.) | - | 0.3 | - | 0.3 | 0.3 | 0.3 | - | 0.3 |
| Magnesium Stearate (general, Taihei Chemicals Ltd.) | 2 | 1.5 | 2 | 1.5 | 1.5 | 1.5 | 2 | 1.5 |
| Total in Uncoated Tablet | 100 | 100 | 100 | 100 | 100 | 100 | 100 | 100 |
| OPADRY(R) | 5 | 5 | 5 | 3.67 | 3.67 | 3.67 | - | - |
| Total | 105 | 105 | 105 | 103.67 | 103.67 | 103.67 | 100 | 100 |

| | | | | | | | | |
|---|---|---|---|---|---|---|---|---|
| 1) product sifted through 200 mesh 2) product pulverized using fine impact mill | | | | | | | | |

### (9) Evaluation Method and Results

The tablets of each of Examples 1 to 6 and Comparative Examples 1 and 2 were stored under open conditions of 25°C/75% RH/1 day, and then, their appearance was visually checked to count the number of tablets, out of 100 tablets, having changed appearance.

Results are shown in Table 2.

When the tablets were produced without treating citric acid hydrate (Examples 1 and 2), fine dimples having a size of about 100 to 300 µm were found on the surface of the tablets after storage under open conditions of 25°C/75% RH/1 day.

On the other hand, when the same production method was employed but citric acid hydrate was not contained in the formulation, the appearance was not changed after the storage under open conditions (Example 3). Besides, even when the same production methods as those of Examples 1 and 2 were employed, the appearance was not changed if the tablets were uncoated (Comparative Examples 1 and 2).

It was presumed, based on these results, that fine dimples formed during storage at high humidity are a phenomenon peculiar to film-coated tablets, and are derived from citric acid anhydride or citric acid hydrate (citric acid hydrate, in particular).

When premixing of citric acid hydrate was additionally performed and the intensity of sifting a mixed powder using a Comil was increased to increase dispersibility of the citric acid hydrate in the mixed powder (Example 5), an effect of suppressing appearance change was not exhibited.

On the other hand, when citric acid hydrate was sifted (Example 4) or pulverized (Example 6), the effect of suppressing appearance change was remarkably exhibited. In comparison between Examples 5 and 6, the appearance change was remarkably suppressed only when the citric acid hydrate was pulverized, even if the production methods were the same. It was revealed, based on these results, that the appearance change caused during storage at high humidity can be suppressed by removing coarse particles of citric acid hydrate by sifting, pulverization, or the like.

**[Table 2]**

| | Example 1 | Example 2 | Example 3 | Example 4 | Example 5 | Example 6 | Comparative Example 1 | Comparative Example 2 |
|---|---|---|---|---|---|---|---|---|
| Citric Acid Hydrate (wt%) | 1.5 | 2.81 | 0 | 1.5 | 1.5 | 1.5 | 1.5 | 2.81 |
| Citric Acid Treatment in Production | - | - | No Citric Acid | Sifted | Dispersibility Improved | Pulverized and Dispersibility Improved | (Uncoated Tablet) | (Uncoated Tablet) |
| Number of Tablets having Appearance Change under Open Conditions of 25°C/75% RH/1 day (/100 tablets) | 32 | 30 | 0 | 0 | 46 | 2 | 0 | 0 |

### (Examples 4, and 7 to 9) Influence of Particle Size Distribution of Citric Acid Hydrate on Effect of Suppressing Appearance Change

### (1) Example 7

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 25 minutes.

### (Sifting Citric Acid Hydrate)

Citric acid hydrate was manually sifted through a 140 mesh (having an opening of 106 µm) to obtain a citric acid hydrate sifted powder.

### (Mixing/sifting)

D-mannitol, carmellose calcium, the citric acid hydrate sifted powder, the dl-α-tocopherol mixed triturated powder, and magnesium aluminometasilicate were weighed out in the blending ratio shown in Table 4, and the resultant mixture was mixed using a V-type mixer (2 L) for 10 minutes at a rotation speed of 39 rpm.

The resultant was sifted using a Comil (U-5, φ1.143, QUADRO) at 1560 rpm to obtain a sifted powder.

Subsequently, magnesium stearate was weighed out in the blending ratio shown in Table 4 to be added to the sifted powder, and the resultant was mixed using a V-type mixer (2 L) for 7 minutes at a rotation speed of 39 rpm.

### (Compressing)

The resultant was molded using a tablet press (Vela 2, Kikusui Seisakusho Ltd.) with the tablet mass set to 300 mg at a compression pressure of about 15 kN to obtain uncoated tablets (oblong tablet, 12.1 x 6.4 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated with a coating machine (Hicoater FZ 20, Freund Corp.) at a charge air temperature of 70°C, a charge air flow rate of 0.5 m³/min, a spray rate of about 2 g/min, a pan rotation speed of 20 rpm, and an exhaust gas drying end point of about 54°C to obtain coated tablets.

### (2) Examples 8 and 9

In Examples 8 and 9, respective ingredients and their contents shown in Table 4 were employed in the preparation method of Example 7 to prepare coated tablets.

Citric acid hydrate was, however, sifted under the conditions shown in Table 3.

**[Table 3]**

| | Mesh used |
|---|---|
| Example 8 | 100 Mesh (having opening of 150 µm) |
| Example 9 | 80 Mesh (having opening of 180 µm) |

**[Table 4]**

| | Composition (wt%/uncoated tablet) | | | |
|---|---|---|---|---|
| Ingredients | Example 4 | Example 7 | Example 8 | Example 9 |
| Mirogabalin Besylate (in terms of mirogabalin) | 8.78 (5) | - | - | - |
| D-mannitol (Parteck M100, Merck, Mannogem EZ, SPI Pharma) | 76.92 | 85.70 | 85.70 | 85.70 |
| Carmellose Calcium (E.C.G-505, Gotoku Chemical Co., Ltd.) | 10 | 10 | 10 | 10 |
| dl-α-Tocopherol (Mitsubishi Chemical Foods Corp.) | 0.1 | 0.1 | 0.1 | 0.1 |
| Crystalline Cellulose (Ceolus UF-702, Asahi Kasei Corp.) | 0.9 | 0.9 | 0.9 | 0.9 |
| Citric Acid Hydrate (Merck) | 1.5³⁾ | 1.5⁴⁾ | 1.5⁵⁾ | 1.5⁶⁾ |
| Magnesium Aluminometasilicate (Neusilin UFL2, US2, Fuji Chemical Industries, Co., Ltd.) | 0.3 | 0.3 | 0.3 | 0.3 |
| Magnesium Stearate (general, Taihei Chemicals Ltd.) | 1.5 | 1.5 | 1.5 | 1.5 |
| Total in Uncoated Tablet | 100 | 100 | 100 | 100 |
| OPADRY(R) | 3.67 | 3.67 | 3.67 | 3.67 |
| Total | 103.67 | 103.67 | 103.67 | 103.67 |

| | | | | |
|---|---|---|---|---|
| 3) product sifted through 200 mesh 4) product sifted through 140 mesh 5) product sifted through 100 mesh 6) product sifted through 80 mesh | | | | |

### (3) Evaluation Method and Results

The tablets of each of Examples 4 and 7 to 9 were stored under open conditions of 25°C/75% RH/1 day or 1 week, and then, their appearance was visually checked to count the number, out of 100 tablets, of tablets having appearance change.

The particle size distribution of the citric acid hydrate sifted powder used was measured by a laser diffraction method (HELOS system, Sympatec GmbH).

Results of the appearance evaluation are shown in Table 5, and measurement results of the particle size distribution of the citric acid hydrate sifted powders used are shown in Table 6.

In a preparation produced by using citric acid hydrate having a particle size distribution x99 of less than 210 µm from which coarse particles had been removed, the appearance was not changed even through storage at high humidity (Example 4, Example 7 and Example 8).

In particular, in a preparation produced by using citric acid hydrate having a particle size distribution x99 of less than 200 µm, or having a particle size distribution x99 of less than 150 µm, favorable results were obtained (Example 4 and Example 7).

On the other hand, in a preparation produced by using citric acid hydrate having a particle size distribution x99 of over 250 µm in which coarse particles were contained, fine dimples having a size of about 100 to 300 µm were formed during storage at high humidity (Example 9).

It was revealed, based on these results, that the appearance change caused during storage at high humidity is derived from the size of coarse particles of citric acid hydrate.

Besides, it was revealed that the appearance change caused during storage at high humidity can be suppressed by removing coarse particles of citric acid hydrate having a size equivalent to or larger than the size of the fine dimples.

**[Table 5]**

| | Example 4 | Example 7 | Example 8 | Example 9 |
|---|---|---|---|---|
| Number of Tablets having Appearance Change under Open Conditions of 25°C/75% RH/1 day (/100 tablets) | 0 | 0 | 0 | 24 |
| Number of Tablets having Appearance Change under Open Conditions of 25°C/75% RH/1 week (/100 tablets) | 0 | 0 | 1 | - |
| Number of Tablets having Appearance Change under Open Conditions of 25°C/75% RH/6 months (/100 tablets) | 0 | 0 | - | - |

**[Table 6]**

| | Example 4 | Example 7 | Example 8 | Example 9 | Untreated |
|---|---|---|---|---|---|
| Particle Size Distribution x50 (µm) | 24.2 | 25.1 | 26.2 | 28.4 | 31.3 |
| Particle Size Distribution x90 (µm) | 67.8 | 73.3 | 100.1 | 116.2 | 127.8 |
| Particle Size Distribution x99 (µm) | 112.6 | 125.6 | 207.1 | 271.4 | 320.7 |

### (Reference Examples 1 to 7) Examination on Stabilizing Effect depending on Blending Amounts of α-Tocopherol and Citric Acid Hydrate

### (1) Reference Example 1

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 5 minutes.

### (Mixing/sifting)

Mirogabalin besylate, D-mannitol, carmellose calcium, citric acid hydrate, the dl-α-tocopherol mixed triturated powder, and magnesium aluminometasilicate were weighed out in the blending ratio shown in Table 7, and the resultant mixture was mixed using a V-type mixer (5 L) for 10 minutes at a rotation speed of 34 rpm.

The resultant was sifted using a Comil (U-5, φ1.143, QUADRO) at 1560 rpm to obtain a sifted powder.

Subsequently, magnesium stearate was weighed out in the blending ratio shown in Table 7 to be added to the sifted powder, and the resultant was mixed using a V-type mixer (5 L) for 7 minutes at a rotation speed of 34 rpm.

### (Compressing)

The resultant was molded using a tablet press (Vela 5, Kikusui Seisakusho Ltd.) with the tablet mass set to 100 mg at a compression pressure of about 9 kN to obtain uncoated tablets (containing mirogabalin in an amount of 2.5% by weight with respect to the uncoated tablet, oblong tablet, 8.4 x 4.4 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated using a coating machine (Hicoater LABO 30, Freund Corp.) at a charge air temperature of 70°C, a charge air flow rate of 0.8 m³/min, a spray rate of about 8 g/min, a pan rotation speed of 20 rpm, and an exhaust gas drying end point of about 60°C to obtain coated tablets.

### (2) Reference Examples 2 to 5

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 5 minutes.

### (Mixing/sifting)

Mirogabalin besylate, D-mannitol, carmellose calcium, carmellose, citric acid hydrate, magnesium aluminometasilicate, and the dl-α-tocopherol mixed triturated powder were weighed out in the blending ratio shown in Table 7, and the resultant mixture was mixed using a V-type mixer (5 L) for 10 minutes at a rotation speed of 34 rpm.

The resultant was sifted using a Comil (QC-194S, φ1.143, QUADRO) at 600 rpm to obtain a sifted powder.

Subsequently, magnesium stearate was weighed out in the blending ratio shown in Table 7 to be added to the sifted powder, and the resultant was mixed using a V-type mixer (5 L) for 7 minutes at a rotation speed of 34 rpm.

### (Compressing)

The resultant was molded using a tablet press (Virgo, Kikusui Seisakusho Ltd.) with the tablet mass set to 200 mg at a compression pressure of about 10 kN to obtain uncoated tablets (containing mirogabalin in an amount of 2.5% by weight with respect to the uncoated tablet, oblong tablet, 10.6 x 5.6 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated using a coating machine (Hicoater LABO 30, Freund Corp.) at a charge air temperature of 70°C, a charge air flow rate of 0.8 m³/min, a spray rate of about 8 g/min, a pan rotation speed of 20 rpm, and an exhaust gas drying end point of about 60°C to obtain a coated tablet.

### (3) Reference Example 6

### (Trituration Mixing)

A dl-α-tocopherol mixed triturated powder was obtained by mixing 80.0 g of dl-α-tocopherol and 720.0 g of crystalline cellulose using a high-speed agitation granulator (VG-5L, Powrex Corp.) at an agitator rotation speed of 280 rpm and a chopper rotation speed of 3000 rpm for 25 minutes.

### (Mixing/sifting)

Mirogabalin besylate, D-mannitol, carmellose calcium, citric acid hydrate, the dl-α-tocopherol mixed triturated powder, and magnesium aluminometasilicate were weighed out in the blending ratio shown in Table 7, and the resultant mixture was mixed using a V-type mixer (2 L) for 10 minutes at a rotation speed of 39 rpm.

The resultant was sifted using a Comil (U-5, φ1.143, QUADRO) at 1560 rpm to obtain a sifted powder.

Subsequently, magnesium stearate was weighed out in the blending ratio shown in Table 7 to be added to the sifted powder, and the resultant was mixed using a V-type mixer (2 L) for 7 minutes at a rotation speed of 39 rpm.

### (Compressing)

The resultant was molded using a tablet press (Vela 2, Kikusui Seisakusho Ltd.) with the tablet mass set to 100 mg at a compression pressure of about 9 kN to obtain uncoated tablets (containing mirogabalin in an amount of 2.5% by weight with respect to the uncoated tablet, round tablet, 6.5 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated using a coating machine (Hicoater FZ 20, Freund Corp.) at a charge air temperature of 70°C, a charge air flow rate of 0.5 m³/min, a spray rate of about 2 g/min, a pan rotation speed of 20 rpm, and an exhaust gas drying end point of about 54°C to obtain coated tablets.

### (4) Reference Example 7

### (Trituration Mixing)

A dl-α-tocopherol mixed triturated powder was obtained by mixing 120.0 g of dl-α-tocopherol and 680.0 g of crystalline cellulose using a high speed agitation granulator (VG-5L, Powrex Corp.) at an agitator rotation speed of 280 rpm and a chopper rotation speed of 3000 rpm for 25 minutes, and mixing 16.23 g of the thus obtained mixed powder and 33.77 g of crystalline cellulose in a mortar for 5 minutes.

### (Mixing/sifting)

Mirogabalin besylate, D-mannitol, carmellose calcium, citric acid hydrate, the dl-α-tocopherol mixed triturated powder, and magnesium aluminometasilicate were weighed out in the blending ratio shown in Table 7, and the resultant mixture was mixed using a V-type mixer (5 L) for 10 minutes at a rotation speed of 34 rpm.

The resultant was sifted using a Comil (U-5, φ1.143, QUADRO) at 1560 rpm to obtain a sifted powder.

Subsequently, magnesium stearate was weighed out in the blending ratio shown in Table 7 to be added to the sifted powder, and the resultant was mixed using a V-type mixer (5 L) for 7 minutes at a rotation speed of 34 rpm.

### (Compressing)

The resultant was molded using a tablet press (Vela 2, Kikusui Seisakusho Ltd.) with the tablet mass set to 100 mg at a compression pressure of about 9 kN to obtain uncoated tablets (containing mirogabalin in an amount of 2.5% by weight with respect to the uncoated tablet, round tablet, 6.5 mm).

### (Coating)

A stirrer (MAZELA Z, Tokyo Rikakikai Co., Ltd.) was used to disperse OPADRY (R) in purified water (12.5 w/w%) to obtain a coating liquid.

The uncoated tablets were coated using a coating machine (Dria Coater 300, Powrex Corp.) at a charge air temperature of 70°C, a charge air flow rate of 1.2 m³/min, a spray rate of about 7 g/min, a pan rotation speed of 20 rpm, and an exhaust gas drying end point of about 60°C to obtain coated tablets.

**[Table 7]**

| | Composition (wt%/uncoated tablet) | | | | | | |
|---|---|---|---|---|---|---|---|
| Ingredients | Reference Example 1 | Reference Example 2 | Reference Example 3 | Reference Example 4 | Reference Example 5 | Reference Example 6 | Reference Example 7 |
| Mirogabalin Besylate (in terms of mirogabalin) | 4.39 (2.5) | 4.39 (2.5) | 4.39 (2.5) | 4.39 (2.5) | 4.39 (2.5) | 4.39 (2.5) | 4.39 (2.5) |
| D-mannitol (Parteck M100, Merck, Mannogem EZ, SPI Pharma) | 81.31 | 77.83 | 76.61 | 79.11 | 76.61 | 81.31 | 80.69 |
| Carmellose Calcium (E.C.G-505, Gotoku Chemical Co., Ltd.) | 10 | 10 | 10 | 10 | 10 | 10 | 10 |
| dl-α-Tocopherol (Mitsubishi Chemical Foods Corp.) | 0.1 | 0.3 | 0.1 | 0.2 | 0.5 | 0.005 | 0.075 |
| Citric Acid Hydrate (Merck) | 1.5 | 1.9 | 2.9 | 2.8 | 2.5 | 1.5 | 1.125 |
| Crystalline Cellulose (Ceolus UF-702, Asahi Kasei Corp.) | 0.9 | 2.5 | 1.2 | 1.7 | 4.5 | 0.995 | 0.925 |
| Carmellose (NS-300, Gotoku Chemical Co., Ltd.) | - | 1.47 | 3.0 | - | - | - | - |
| Magnesium Aluminometasilicate (Neusilin UFL2, US2, Fuji Chemical Industries, Co., Ltd.) | 0.3 | 0.15 | 0.3 | 0.3 | - | 0.3 | 0.3 |
| Magnesium Stearate (general, Taihei Chemicals Ltd.) | 1.5 | 1.5 | 1.5 | 1.5 | 1.5 | 1.5 | 1.5 |
| Total in Uncoated Tablet | 100 | 100 | 100 | 100 | 100 | 100 | 100 |
| OPADRY (R) | 5 | 5 | 5 | 5 | 5 | 5 | 5 |
| Total | 105 | 105 | 105 | 105 | 105 | 105 | 105 |

### (5) Evaluation Method and Results

After the tablets of Reference Examples 1 to 7 were left under open conditions of 25°C/75% RH/12 weeks, principal decomposition products were measured by UHPLC (1290 Infinity, Agilent) under the following analysis conditions. It is noted that open conditions of 25°C/75% RH/3 months were employed for only Reference Example 6 to obtain a result.

### (Analysis Conditions for UHPLC)

Measurement wavelength: 215 nm
Column: Sunsell C18 (2.1 mm ID x 100 mm, 2.6 µm, Chromanik Technologies Inc.)
Guard column: Security Guard ULTRA C18 (2.1 mm ID, Phenomenex Inc.)
Cleanup column: Ghost Trap DS (7.6 mm ID x 30 mm, Shimadzu Corporation)
Column temperature: 45°C
Mobile phase A: 0.01 mol/L diammonium hydrogen phosphate buffer (pH 6.2)
Mobile phase B: methanol/acetonitrile/0.01 mol/L diammonium hydrogen phosphate buffer (pH 6.2) mixture (9:3:4)
Analysis time: 35 min
Injection amount: 3 µL
Sample cooler temperature: constant temperature around 6°C

### (Relative Retention Time of Decomposition Products A and B with respect to Mirogabalin)

Decomposition product A: maximum value around 0.3
Decomposition product B: 2.0 to 2.1

Results are shown in Table 8 (amounts of decomposition product A and decomposition product B, %). As a result, when 0.005 to 0.5% by weight of dl-α-tocopherol and 1.125 to 2.9% by weight of citric acid hydrate were blended in combination, production amounts of these decomposition products could be suppressed to equal to or lower than a reference value "0.2%, that is, a threshold value for requiring structure determination of an impurity" (0.15 or more and less than 0.25) described in "Shin-yukoseibun ganyu iyakuhin no uchi seizai no fujunbutsu ni kansuru gaidorain no kaitei nitsuite (Revision of guidelines on impurities in pharmaceuticals containing new active ingredients) (PMSB/ELD Notification No. 0624001, dated June 24, 2003)".

**[Table 8]**

| | Reference Example 1 | Reference Example 2 | Reference Example 3 | Reference Example 4 | Reference Example 5 | Reference Example 6 | Reference Example 7 |
|---|---|---|---|---|---|---|---|
| dl-α-Tocopherol (wt%) | 0.1 | 0.3 | 0.1 | 0.2 | 0.5 | 0.005 | 0.075 |
| Citric Acid Hydrate (wt%) | 1.5 | 1.9 | 2.9 | 2.8 | 2.5 | 1.5 | 1.125 |
| Decomposition Product A (%) obtained under Open Conditions of 25°C/75% RH/12 weeks | <0.05 | 0.06 | 0.07 | 0.07 | 0.10 | 0.05 | 0.08 |
| Decomposition Product B (%) obtained under Open Conditions of 25°C/75% RH/12 weeks | 0.18 | 0.17 | 0.12 | 0.12 | 0.17 | 0.22 | 0.21 |

## Claims

1. A film-coated tablet obtained by film coating an uncoated tablet comprising citric acid anhydride having a particle size distribution x99 of less than 210 µm or citric acid hydrate having a particle size distribution x99 of less than 210 µm, and α-tocopherol, wherein particle size is measured by laser diffraction.

2. A film-coated tablet obtained by film coating uncoated tablets comprising:
mirogabalin or a pharmaceutically acceptable salt thereof,
citric acid anhydride having a particle size distribution x99 of less than 210 µm or citric acid hydrate having a particle size distribution x99 of less than 210 µm, wherein particle size is measured by laser diffraction, and
α-tocopherol.

3. The film-coated tablet according to claim 2, wherein mirogabalin or a pharmaceutically acceptable salt thereof is mirogabalin besylate.

4. The film-coated tablet according to any one of claims 1 to 3, wherein the content of the citric acid anhydride having a particle size distribution x99 of less than 210 µm or the citric acid hydrate having a particle size distribution x99 of less than 210 µm is, in terms of citric acid hydrate, 1.0 to 5.0% by weight based on 100% by weight of the uncoated tablet.

5. The film-coated tablet according to any one of claims 1 to 3, wherein the content of the citric acid anhydride having a particle size distribution x99 of less than 210 µm or the citric acid hydrate having a particle size distribution x99 of less than 210 µm is, in terms of citric acid hydrate, 1.125 to 2.9% by weight based on 100% by weight of the uncoated tablet.

6. The film-coated tablet according to any one of claims 1 to 5, wherein the particle size distribution x99 of the citric acid anhydride or the citric acid hydrate is less than 150 µm.

7. The film-coated tablet according to any one of claims 1 to 6, wherein the content of α-tocopherol is 0.005 to 1.0% by weight based on 100% by weight of the uncoated tablet.

8. The film-coated tablet according to any one of claims 1 to 6, wherein the content of α-tocopherol is 0.005 to 0.5% by weight based on 100% by weight of the uncoated tablet.

9. The film-coated tablet according to any one of claims 3 to 7, wherein the content of mirogabalin besylate is, in terms of mirogabalin, 1.0 to 5.0% by weight based on 100% by weight of the uncoated tablet.

10. The film-coated tablet according to any one of claims 1 to 9, further comprising 75 to 85% by weight of D-mannitol having an average particle size of 120 µm or less based on 100% by weight of the uncoated tablet.

11. The film-coated tablet according to any one of claims 1 to 10, further comprising 5 to 15% by weight carmellose calcium based on 100% by weight of the uncoated tablet.

12. A method for producing a film-coated tablet obtained by film coating an uncoated tablet comprising mirogabalin or a pharmaceutically acceptable salt thereof, the method comprising:
(i) a step of producing an uncoated tablet comprising mirogabalin or a pharmaceutically acceptable salt thereof, citric acid anhydride having a particle size distribution x99 of less than 210 µm or citric acid hydrate having a particle size distribution x99 of less than 210 µm, and α-tocopherol, wherein particle size is measured by laser diffraction; and
(ii) a step of film coating the uncoated tablet obtained in (i).

## Patentansprüche

1. Filmüberzogene Tablette, erhalten durch Filmüberzug einer unbeschichteten Tablette, umfassend Zitronensäureanhydrid mit einer Teilchengrößenverteilung x99 von weniger als 210 µm oder Zitronensäurehydrat mit einer Teilchengrößenverteilung x99 von weniger als 210 µm und α-Tocopherol, wobei die Teilchengröße durch Laserbeugung gemessen wird.

2. Filmüberzogene Tablette, erhalten durch Filmüberzug von unbeschichteten Tabletten, umfassend:
Mirogabalin oder ein pharmazeutisch verträgliches Salz davon,
Zitronensäureanhydrid mit einer Teilchengrößenverteilung x99 von weniger als 210 µm oder Zitronensäurehydrat mit einer Teilchengrößenverteilung x99 von weniger als 210 µm, wobei die Teilchengröße durch Laserbeugung gemessen wird, und
α-Tocopherol.

3. Filmüberzogene Tablette nach Anspruch 2, wobei Mirogabalin oder ein pharmazeutisch verträgliches Salz davon Mirogabalinbesylat ist.

4. Filmüberzogene Tablette nach einem der Ansprüche 1 bis 3, wobei der Gehalt an Zitronensäureanhydrid mit einer Teilchengrößenverteilung x99 von weniger als 210 µm oder Zitronensäurehydrat mit einer Teilchengrößenverteilung x99 von weniger als 210 µm, bezogen auf Zitronensäurehydrat, 1,0 bis 5,0 Gew.-% beträgt, bezogen auf 100 Gew.-% der unbeschichteten Tablette.

5. Filmüberzogene Tablette nach einem der Ansprüche 1 bis 3, wobei der Gehalt an Zitronensäureanhydrid mit einer Teilchengrößenverteilung x99 von weniger als 210 µm oder Zitronensäurehydrat mit einer Teilchengrößenverteilung x99 von weniger als 210 µm, bezogen auf das Citronensäurehydrat, 1,125 bis 2,9 Gew.-% beträgt, bezogen auf 100 Gew.-% der unbeschichteten Tablette.

6. Filmüberzogene Tablette nach einem der Ansprüche 1 bis 5, wobei die Teilchengrößenverteilung x99 des Zitronensäureanhydrids oder des Zitronensäurehydrats weniger als 150 µm beträgt.

7. Filmüberzogene Tablette nach einem der Ansprüche 1 bis 6, wobei der Gehalt an α-Tocopherol 0,005 bis 1,0 Gew.-% beträgt, bezogen auf 100 Gew.-% der unbeschichteten Tablette.

8. Filmüberzogene Tablette nach einem der Ansprüche 1 bis 6, wobei der Gehalt an α-Tocopherol 0,005 bis 0,5 Gew.-% beträgt, bezogen auf 100 Gew.-% der unbeschichteten Tablette.

9. Filmüberzogene Tablette nach einem der Ansprüche 3 bis 7, wobei der Gehalt an Mirogabalinbesylat, bezogen auf Mirogabalin, 1,0 bis 5,0 Gew.-% beträgt, bezogen auf 100 Gew.-% der unbeschichteten Tablette

10. Filmüberzogene Tablette nach einem der Ansprüche 1 bis 9, ferner umfassend 75 bis 85 Gew.-% D-Mannit mit einer durchschnittlichen Teilchengröße von 120 µm oder weniger, bezogen auf 100 Gew.-% der unbeschichteten Tablette.

11. Filmüberzogene Tablette nach einem der Ansprüche 1 bis 10, ferner umfassend 5 bis 15 Gew.-% Carmellose-Calcium, bezogen auf 100 Gew.-% der unbeschichteten Tablette.

12. Verfahren zur Herstellung einer filmüberzogenen Tablette, erhalten durch Filmüberzug einer unbeschichteten Tablette, umfassend Mirogabalin oder ein pharmazeutisch verträgliches Salz davon, wobei das Verfahren umfasst:
(i) einen Schritt zur Herstellung einer unbeschichteten Tablette, umfassend Mirogabalin oder ein pharmazeutisch verträgliches Salz davon, Zitronensäureanhydrid mit einer Teilchengrößenverteilung x99 von weniger als 210 µm oder Zitronensäurehydrat mit einer Teilchengrößenverteilung x99 von weniger als 210 µm und α-Tocopherol, wobei die Teilchengröße durch Laserbeugung gemessen wird; und
(ii) einen Schritt des Filmüberzugs der in (i) erhaltenen unbeschichteten Tablette.

## Revendications

1. Comprimé pelliculé obtenu par enrobage par film d'un comprimé non enrobé comprenant de l'acide citrique anhydre ayant une répartition granulométrique x99 de moins de 210 µm ou de l'acide citrique hydraté ayant une répartition granulométrique x99 de moins de 210 µm, et de l'α-tocophérol, dans lequel la taille de particule est mesurée par diffraction laser.

2. Comprimé pelliculé obtenu par enrobage par film de comprimés non enrobés comprenant :
de la mirogabaline ou un sel pharmaceutiquement acceptable de celle-ci,
de l'acide citrique anhydre ayant une répartition granulométrique x99 de moins de 210 µm ou de l'acide citrique hydraté ayant une répartition granulométrique x99 de moins de 210 µm, dans lequel la taille de particule est mesurée par diffraction laser, et
de l'α-tocophérol.

3. Comprimé pelliculé selon la revendication 2, dans lequel la mirogabaline ou un sel pharmaceutiquement acceptable de celle-ci est du bésylate de mirogabaline.

4. Comprimé pelliculé selon l'une quelconque des revendications 1 à 3, dans lequel la teneur en acide citrique anhydre ayant une répartition granulométrique x99 de moins de 210 µm ou en acide citrique hydraté ayant une répartition granulométrique x99 de moins de 210 µm est, en termes d'acide citrique hydraté, de 1,0 à 5,0 % en poids sur la base de 100 % en poids du comprimé non enrobé.

5. Comprimé pelliculé selon l'une quelconque des revendications 1 à 3, dans lequel la teneur en acide citrique anhydre ayant une répartition granulométrique x99 de moins de 210 µm ou en acide citrique hydraté ayant une répartition granulométrique x99 de moins de 210 µm est, en termes d'acide citrique hydraté, de 1,125 à 2,9 % en poids sur la base de 100 % en poids du comprimé non enrobé.

6. Comprimé pelliculé selon l'une quelconque des revendications 1 à 5, dans lequel la répartition granulométrique x99 de l'acide citrique anhydre ou de l'acide citrique hydraté est de moins de 150 µm.

7. Comprimé pelliculé selon l'une quelconque des revendications 1 à 6, dans lequel la teneur en α-tocophérol est de 0,005 à 1,0 % en poids sur la base de 100 % en poids du comprimé non enrobé.

8. Comprimé pelliculé selon l'une quelconque des revendications 1 à 6, dans lequel la teneur en α-tocophérol est de 0,005 à 0,5 % en poids sur la base de 100 % en poids du comprimé non enrobé.

9. Comprimé pelliculé selon l'une quelconque des revendications 3 à 7, dans lequel la teneur en bésylate de mirogabaline est, en termes de mirogabaline, de 1,0 à 5,0 % en poids sur la base de 100 % en poids du comprimé non enrobé.

10. Comprimé pelliculé selon l'une quelconque des revendications 1 à 9, comprenant en outre 75 à 85 % en poids de D-mannitol ayant une taille moyenne de particule de 120 µm ou moins sur la base de 100 % en poids du comprimé non enrobé.

11. Comprimé pelliculé selon l'une quelconque des revendications 1 à 10, comprenant en outre 5 à 15 % en poids de carmellose calcique sur la base de 100 % en poids du comprimé non enrobé.

12. Procédé de fabrication d'un comprimé pelliculé, obtenu par enrobage par film d'un comprimé non enrobé, comprenant de la mirogabaline ou un sel pharmaceutiquement acceptable de celle-ci, le procédé comprenant :
(i) une étape de fabrication d'un comprimé non enrobé comprenant de la mirogabaline ou un sel pharmaceutiquement acceptable de celle-ci, de l'acide citrique anhydre ayant une répartition granulométrique x99 de moins de 210 µm ou de l'acide citrique hydraté ayant une répartition granulométrique x99 de moins de 210 µm, et de l'α-tocophérol, dans lesquels la taille de particule est mesurée par diffraction laser ; et
(ii) une étape d'enrobage par film du comprimé non enrobé obtenu à l'étape (i).
